# EUROPÄISCHE PATENTSCHRIFT

(11) **EP 0 863 906 B1**
(45) Veröffentlichungstag und Bekanntmachung des Hinweises auf die Patenterteilung: **12.02.2003**
(21) Anmeldenummer: 96934718.6
(22) Anmeldetag: 17.10.1996
(51) Int. Cl.: C07D 493/10, G01N 33/533, C07F 7/18, C07F 9/38, C07J 1/00

(54) **HETEROCYCLISCHE DIOXETAN-SUBSTRATE, VERFAHREN ZUR HERSTELLUNG UND VERWENDUNG**
HETEROCYCLIC DIOXETHANE SUBSTRATES, PROCESS FOR THEIR PREPARATION AND THEIR USE
SUBSTRATS HETEROCYCLIQUES EN DIOXETRANE, PROCEDE PERMETTANT DE LES PREPARER ET LEUR UTILISATION

(30) Priorität: 18.10.1995 DE 19538708
(43) Veröffentlichungstag der Anmeldung: 16.09.1998
(73) Patentinhaber: Roche Diagnostics GmbH, 68298 Mannheim (DE)
(72) Erfinder: HEINDL, Dieter, D-81549 München (DE); JOSEL, Hans-Peter, D-82362 Weilheim (DE); VON DER ELTZ, Herbert, D-82362 Weilheim (DE); HERRMANN, Rupert, D-82362 Weilheim (DE); HÖLTKE, Hans-Joachim, D-82377 Penzberg (DE); BECKERT, Rainer, D-07751 Jena-Wogau (DE); WEISS, Dieter, D-07743 Jena (DE); ADAM, Waldemar, D-97074 Würzburg (DE)
(86) Internationale Anmeldenummer: EP9604506
(87) Internationale Veröffentlichungsnummer: WO97014696

(56) Entgegenhaltungen:
- EP-A- 0 254 051
- EP-A- 0 352 713
- EP-A- 0 379 716
- EP-A- 0 473 984
- WO-A-90/07511
- WO-A-93/20083
- WO-A-94/10258
- DE-A- 4 210 759
- US-A- 5 112 960

## Beschreibung

Gegenstand der Erfindung sind heterocyclische Dioxetan-Substrate, Verfahren zur Herstellung und Verwendung in enzymatischen Analyseverfahren.

Es ist seit längerem bekannt, daß durch die Umsetzung von Luciferin mit Luciferase, Sauerstoff und ATP Oxiluciferin entsteht. Bei dieser Reaktion wird Licht (Wellenlängenmaximum bei 562 nm) als Chemilumineszenz emittiert. Als energiereiches Zwischenprodukt entsteht dabei vermutlich ein Dioxetan (F. McCapra, Chem. Commun. 155 (1968)). Aufgrund dieses Postulats wurden eine Vielzahl von chemilumineszierenden 1,2-Dioxetan-Verbindungen entwickelt.

Zur Stabilisierung der an sich instabilen 1,2-Dioxetane wurde der Adamantyl-Rest beschrieben (EP-A 0 254 051, EP-A 0 352 713, WO 91/03479, WO 90/07511, WO 92/04341, WO 94/10258 sowie dort zitierte Publikationen). Die Chemilumineszenzausbeute entwickelt sich dann mit Zerfall der Dioxetane und ist unter anderem von den Fluoreszenzeigenschaften des gebildeten Emitters abhängig. Die beim Zerfall der üblicherweise verwendeten Dioxetan-Derivate (3-(2'-Spiroadamantan)-4-methoxy-4-(3"-phosphoryloxy) phenyl-1,2-Dioxetan bzw. entsprechende am Adamantylrest halogenierte Dioxetane) gebildeten Phenolate weisen zwar eine gute Fluoreszenzquantenausbeute auf, die Substrate zeigen jedoch eine insgesamt niedrige Chemilumineszenz. Insbesondere wären Dioxetane mit verbesserten Chemilumineszenzausbeuten beim getriggerten Zerfall der Dioxetane wünschenswert.

Aus WO 93/20083 sind stabilisierte Dioxetan-Derivate bekannt, die in Oxyluciferin bzw. Phenylthiazolinderivate als Emitter zerfallen. Diese Dioxetane zerfallen zwar mit einer höheren Lichtausbeute als andere bekannte Dioxetane, sind jedoch synthetisch nur schwer zugänglich.

Aufgabe der vorliegenden Erfindung war es somit, neue 1,2-Dioxetane zur Verfügung zu stellen, welche stabil sind und erst durch Umsetzung mit einem aktivierenden Agens unter Bildung hoher Lichtausbeute zerfallen sowie durch relativ einfache chemische Synthese zugänglich sind. Diese Aufgabe wird gelöst durch eine Verbindung der allgemeinen Formel Ia, 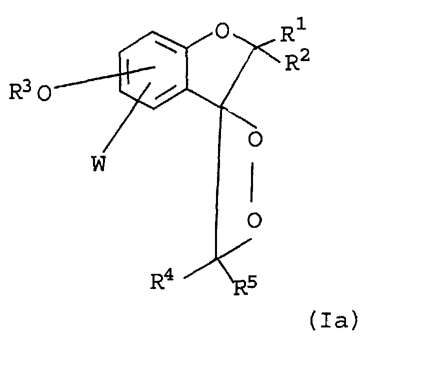 in der
R¹ und R² jeweils gleich oder verschieden sind und Wasserstoff, Niederalkyl C₁ bis C₆, geradkettig oder verzweigt, jeweils oder zusammen eine Phenylgruppe, darstellen mit der Maßgabe, daß höchstens einer der Reste R¹ oder R² Wasserstoff ist,
R³-O- das entsprechende Hydroxyssalz, eine Oxysäure, Phosphat, Aryl- oder Alkylcarboxylester, Alkoxy, Trialkyloxy oder Alkylsilyloxy, Arylsilyloxy, Sulfat, Oxypyranosid oder ein Glyceridyl- bzw. Phosphorylrest oder ein Steroidderiat darstellt,
W Wasserstoff, Halogen oder ein Pseudohalogen wie ein Rhodanid- oder Cyanidrest ist und bevorzugt in ortho-Stellung zur R³-O-Gruppe steht,
mindestens eine der Gruppen R⁴ oder R⁵ Wasserstoff oder eine die Dioxetanstruktur stabilisierende Gruppe darstellt und wobei höchstens eine der Gruppen R⁴ oder R⁵ Wasserstoff ist und die Dioxetanstruktur stabilisierende Gruppe ein Adamantyl-, Phenyl-, Cyclohexyl-, sekundärer oder tertiärer Alkyl-, Polycycloalkyl- bzw. Polycycloarylrest, ein Steroidderivat oder ein Gemisch von letzterem ist.

Die Gruppe -O-R³ kann an jeder Position des Phenylrings lokalisiert sein. Die Gruppe-O-R³ ist vorzugsweise in 5-Stellung angeordnet. Besonders bevorzugt sind für -O-R³ Phosphat sowie die Dimethyl-tertiärbutyl-silyloxygruppe.

Die Zusammensetzung der Aryl- und Alkylreste ist unkritisch. Die Auswahl geeigneter Reste R³ ist jedem Fachmann ohne weiteres möglich. Es muß lediglich Löslichkeit und Abspaltbarkeit von R³ durch die aktivierenden Agenzien möglich sein.

Bevorzugt sind für R¹ und R² Wasserstoff, Methyl-, Ethyl- oder Phenylgruppen bzw. wenn R¹ und R² zusammen eine mit (C)ₘ und m gleich 2 eine Phenylengruppe bedeutet, wobei die Phenyl- bzw. Phenylengruppen beispielsweise durch einen Halogenrest substituiert sein können. Wird beispielsweise Phosphat als -O-R³ verwendet, so kann die Chemilumineszenzreaktion durch Zusatz von alkalischer Phosphatase induziert werden. Bei Einsatz des Galaktosids kann die Chemilumineszenzreaktion durch β-Galaktosidase induziert werden. Wird ein Silyloxyrest als -O-R³ verwendet, so kann die Zugabe von Fluorid die Chemilumineszenz induzieren.

Als Dioxetanstruktur-stabilisierende Gruppen R⁴ und/oder R⁵ geeignet sind Gruppen, welche die Dioxetangruppe vor unkontrollierter Umsetzung schützen. Diese Stabilisierung erfolgt vorzugsweise durch sterische Abschirmung dieser Gruppe. Daher kommen insbesondere kondensierte aliphatische oder aromatische Ringsysteme, die gegebenenfalls durch elektronenziehende Gruppen substituiert sein können, für diesen Zweck in Betracht. Als elektronenziehende Gruppen können beispielsweise Carbonyl oder Halogen eingesetzt werden. Geeignet sind beispielsweise Adamantanyl-, Phenyl-, Cyclohexyl-, sekundäre und tertiäre aliphatische Alkylgruppen (wie z.B. die t-Butylgruppe) in substituierter und unsubstituierter Form. Dabei können R⁴ und R⁵ gleich oder verschieden sein. Im Fall von Phenyl- und/oder Cyclohexylgruppen kommen sowohl einzelne Reste als auch in Form von kondensierten Ringsystemen als Substituenten in Betracht, die gegebenenfalls weitere Struktureinheiten, wie z.B. einen Cyclopentylrest wie in Steroiden, aufweisen können. Entsprechende Reste werden im Rahmen der vorliegenden Erfindung als Polycycloalkyl- bzw. Polycycloarylreste zusammengefaßt. Im Falle des Adamantylrests bzw. beispielsweise eines Steroidylrests ist dieser vorzugsweise so gebunden, daß R⁴ und R⁵ Teile der Ringstruktur bedeuten und demnach überbrückt sind (Formeln Ia' bzw. Ia"):

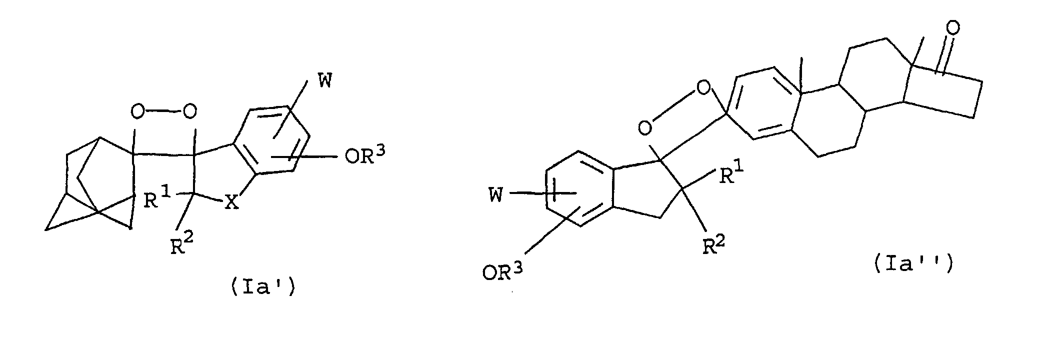

Als besonders geeignet haben sich erfindungsgemäß die Dioxetane der Benzofurane herausgestellt:
3-(7'-t.-Butyldimethylsilyloxy-2',2'-dimethyl-benzofuran-3'-yl)-4-spiro-(tricyclo[3.3.1.1.^{3,7}] yl)-1,2-dioxetan, das Dinatriumsalz des 3-(7'-Phosphoryl-2',2'-dimethyl-benzofuran-3'-yl) -4-spiro-(tricyclo[3.3.1.1.^{3,7}]yl)-1,2-dioxetans, 3-(7'-t.-Butyldimethylsilyloxy-2',2'-dimethyl-benzofuran-3'-yl)-4-spiro-(5"-chlor-tricyclo[3.3.1.1.^{3,7}]yl)-1,2-dioxetan, das Dinatriumsalz des 3-(7'-Phosphoryl-2',2'-dimethyl-benzofuran-3'-yl)-4-spiro-(5"-chlor-tricyclo [3.3.1.1.^{3,7}]yl)-1,2-dioxetans, 3-(5'-t.-Butyldimethylsilyloxy-2',2'-dimethyl-benzofuran-3'-yl)-4-spiro-(5"-chlortricyclo[3.3.1.1.^{3,7}]yl)-1,2-dioxetan, das Dinatriumsalz des 3-(5'-Phosphoryl-2',2'-dimethylbenzofuran-3'-yl)-4-spiro-(5"-chlor-tricyclo[3.3.1.1.^{3,7}]yl)-1,2-dioxetans, 3-(5'-t.-Butyldimethylsilyloxy-6'-chlor-2',2'-dimethyl-benzofuran-3'-yl)-4-spiro -(5"-chlor-tricyclo [3.3.1.1.^{3,7}]yl)-1,2-dioxetan, das Dinatriumsalz des 3-(5'-Phosphoryl-6'-chlor-2',2'-dimethylbenzofuran-3'-yl)-4-spiro-(5"-chlor-tricyclo[3.3.1.1.^{3,7}]yl)-1,2-dioxetans, (4'Ξ)-5"-t.-Butyldimethylsilyloxy-6"-Chlor-2",2"-dimethyl-17-oxo-(3ξO)-dispiro [androsta-1,4-dien-3,3'-[1,2]dioxetan-4',3"-benzofuran oder (4'Ξ)-6"-Chlor-2",2"-dimethyl-17-oxo-(3ξO)-dispiro[androsta-1,4-dien-3,3'-[1,2]dioxetan-4',3"-benzofuran-5"-yl-dinatriumphosphat.

Die Verbindungen der allgemeinen Formel Ia sind neu. Ein Syntheseverfahren für diese Verbindungen ist bisher nicht bekannt.

Gegenstand der Erfindung ist demnach auch ein Verfahren zur Herstellung der Verbindungen der allgemeinen Formel Ia, welches dadurch gekennzeichnet ist, daß man heterocyclische Carbonylverbindungen der allgemeinen Formel IV 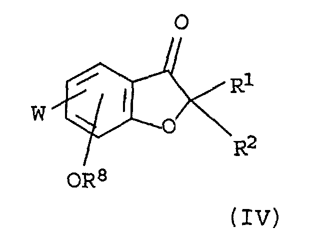 in der R⁸ einen Alkylrest, geradkettig oder verzweigt mit 1 bis 6 C-Atomen, vorzugsweise ein Methylrest, oder ein Wasserstoffatom darstellt, W ein Halogen- oder Pseudohalogenrest, wie beispielsweise SCN oder CN, oder ein Wasserstoffatom darstellt, X Sauerstoff darstellt und die Reste R¹ und R² die oben genannten Bedeutungen haben, mit einer Verbindung der allgemeinen Formel (V) 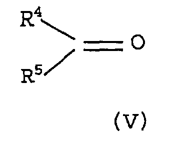 in der R⁴ und R⁵ die oben genannten Bedeutungen haben, unter Wasser und Luftausschluß in Gegenwart von Titantrichlorid und einem Reduktionsmittel umsetzt, das Reaktionsprodukt für R⁸ gleich Alkyl in Gegenwart von beispielsweise Bortrijodid bzw. Natriumethanthiolat dealkyliert bzw., wenn R⁸ Wasserstoff bedeutet, direkt nach einer dem Fachmann geläufigen Methode die abspaltbare Gruppe R³ des Restes -O-R³, der die oben angegebene Bedeutung hat, einführt und anschließend zum Dioxetan dioxigeniert.

Die heterocyclischen Carbonylverbindungen werden nach in der Literatur beschriebenen Methoden hergestellt, vgl. z.B. Tetrahedron 1978, 34, 2035; Helv. Chim. Acta 1972, 55, 1567; J. Agric. Food. Chem. 1968, 300; J. Am. Chem. Soc. 1967, 87, 6527.

Die Umsetzung der Verbindunge IV mit der Verbindung V erfolgt in einem möglichst unpolaren Lösungsmittel, vorzugsweise Tetrahydrofuran oder Dimethoxyethan, in Gegenwart von Titantrichlorid und einem Reduktionsmittel, vorzugsweise Zink/Kupfer oder Lithiumaluminiumhydrid. Als Verbindung V sind insbesondere solche Aldehyde und Ketone geeignet, die das Endprodukt, das Dioxetan stabilisierende Reste R⁴ und R⁵ mit der oben angegebenen Bedeutung aufweisen. Eine solche Stabilisierung kann durch sterische Abschirmung der Dioxetanstruktur erfolgen. Geeignete Ketone und Aldehyde sind demnach beispielsweise Adamantanon, polycyclische Ketone, wie z.B. ein Steroidketon, sekundäre und tertiäre aliphatische Ketone und Aldehyde, wie z.B. die t-Butylketon. Diese Ketone können weitere Substituenten, bevorzugt Chloratome, tragen.

Bei Verbindungen, in denen R⁸ ein Alkylrest, vorzugsweise Methyl bedeutet, erfolgt die Dealkylierung zweckmäßig durch ein Dealkylierungsmittel, vorzugsweise mit Bortrijodid oder Natriumethanthiolat.

Ebenso können die heterocyclischen Ketone IV in die heterocyclischen Thione der allgemeinen Formel VIII überführt werden, in der R⁸ und W, X und R¹ und R² die oben genannten Substituenten darstellen, anschließend mit Diazoverbindungen der allgemeinen Formel IX, in der R⁴ und R⁵ die oben genannten Bedeutungen haben, umgesetzt werden. Die gebildeten Alkene werden für R⁸ gleich Methyl, nach dem oben angegebenen Verfahren dealkyliert. Anschließend erfolgt, wie nachstehend beschrieben, die Einführung der abspaltbaren Gruppe R³ und Dioxygenierung.

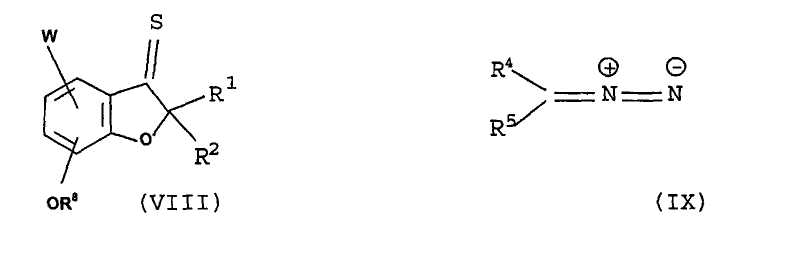

Die Umwandlung der Carbonylfunktion in eine Thiocarbonylfunktion erfolgt durch Umsetzung der heterocyclischen Ketone mit einem Schwefelungsreagenz, bevorzugt Lawesson-Reagenz (2,4-Bis-(4-methoxyphenyl)-1,3-dithia-2,4-diphosphetan-2,4-disulfid) oder Phosphorpentasulfid, im Toluol oder Pyridin.

Die Umsetzung mit Diazokomponenten, beispielsweise mit Di-tertiär-butyldiazomethan oder mit einem Diazosteroid erfolgt bevorzugt in einem unpolaren Lösungsmittel, wie Diethylether. Die Diazokomponente wird durch Oxidation der Hydrazone mit einem Oxidationsmittel, bevorzugt Mangandioxid oder Silberoxid hergestellt. Als Verbindung IX sind vorzugsweise Diazoverbindungen geeignet, die durch die Reste R⁴ und R⁵ das Dioxetan stabilisieren. Eine solche Stabilisierung kann durch sterische Abschirmung der Dioxetanstruktur erfolgen. Geeignete Diazoverbindungen sind demnach beispielsweise Diazoadamantan, Chlordiazoadamantan, Diphenyldiazomethan, polycyclische Diazoverbindungen, wie z.B. eine Diazosteroid, sekundäre und tertiäre aliphatische Ketone und Aldehyde, wie z.B. di-tert.-Butylketon. Diese Diazoverbindungen können weitere Substituenten, bevorzugt Chloratome, tragen.

Die Einführung der abspaltbaren, chemisch labilen Gruppe erfolgt nach dem Fachmann geläufigen Methoden. Derartige Verfahren sind beispielsweise beschrieben in Houben Weyl XII/2.

Die Dioxygenierung erfolgt zweckmäßig durch Umsetzung des Alkens, das entweder in Methylenchlorid oder in Methanol gelöst wird, bei einer Temperatur von ungefähr -30 bis -10°C mit Singulettsauerstoff, der durch Bestrahlung mit sichtbarem Licht unter Verwendung eines Sensitizers (Rose Bengal, Methylenblau, vgl. Tetrahedron Letters 1988, 3137-3140) in situ gebildet wird.

Die Aufreinigung erfolgt insbesondere durch Filtration bzw. andere dem Fachmann geläufige Methoden.

Ein weiterer Gegenstand der Erfindung ist ein Verfahren zur Bestimmung einer Säure, Base eines Salzes, Enzyms, anorganischen oder organischen Katalysators und Elektronendonors durch Umsetzung dieser Verbindung mit einer Verbindung der allgemeinen Formel Ia und Messung des emittierten Lichts als Maß für den Gehalt an der zu bestimmenden Verbindung.

Besonders bevorzugt wird dieses Verfahren verwendet zur Bestimmung von Enzymen, speziell von Markerenzymen in immunologischen Systemen oder zur DNA-Diagnostik mit markierten DNA-Sonden. Bevorzugt wird alkalische Phosphatase bestimmt, wobei die chemisch labile Gruppe R³ Phosphat ist oder β-Galaktosidase, wobei R³ ein Galaktosid ist.

Die Erfindung wird durch folgende Beispiele erläutert:

### Beispiel 1

### 2-(4'-Methoxyphenyl)-4H-1,3-benzoxazin-4-on

Zu einer Suspension von 0.25 mol Salicylamid in 50 ml Xylol werden 2.5 ml Pyridin gegeben. Unter Sieden am Rückfluß (Wasserabscheider) werden innerhalb von 3 h 0.26 mol 4-Methoxybenzoylchlorid gegeben und weitere 3h unter Rückfluß zum Sieden erhitzt. Es werden weitere 2.5 ml Pyridin zugegeben und weitere 8 h erhitzt. Nach dem Abkühlen auf Raumtemp. wird auf 400 ml Isopropanol gegossen und abgesaugt. Der Rückstand wird aus Ethylenglykolmonomethylether umkristallisiert.
Ausbeute 0.2 mol

### Beispiel 2

### 4-Adamantyliden-2-(4'-methoxyphenyl)-4H-1,3-benzoxazin

Zu einer Suspension von 40 mmol Titantrichlorid in 100 ml abs. THF werden unter kräftigen Rühren vorsichtig 21 mmol Lithiumaluminiumhydrid gegeben. Nach einstündigem Rühren bei Raumtemp. wird unter Eiskühlung eine Mischung von 15 mmol 2-(4'-Methoxyphenyl)-4H-1,3 benzoxazin-4-on und 11 mmol Adamantanon gegeben. Es wird 60 min unter Rückfluß zum Sieden erhitzt. Nach Abkühlen auf Raumtemp. wird aur 200 ml Wasser gegossen. Die Mischung wird zweimal mit je 200 ml Essigester ausgeschüttelt. Die vereinigten organischen Phasen werden einmal mit Wasser gewaschen und abgetrennt. Nach Trocknen über Natriumsulfat wird filtriert und das Lösungsmittel abdestilliert. Der ölige Rückstand wird säulenchromatographisch (Kieselgel: Essigester/Petroläther 1 : 9) getrennt.
Ausbeute 6 mmol

### Beispiel 3

### 4-Adamantyliden-2-(4'-hydroxyphenyl)-4H-1,3-benzoxazin

Unter Rühren wird bei - 25 °C unter Luft und Wasserausschluß eine Lösung von 6 mmol 4-Adamantyliden-2-(4'-methoxyphenyl)-4H-1,3-benzoxazin in 20 ml abs. Methylenchlorid zu einer Lösung von 12 mmol Bortrijodid getropft. Die Mischung wird innerhalb einer Stunde auf Raumtemp. erwärmt und weitere 4 h gerührt. Nach Versetzen mit 50 ml Wasser wird die organische Phase abgetrennt und über Natriumsulfat getrocknet. Das Lösungsmittel wird abdestilliert. Der ölige Rückstand wird säulenchromatographisch (Kieselgel: Essigester/Petroläther 1 : 3) getrennt.
Ausbeute 3.2 mmol

### Beispiel 4

### 4-Adamantyliden-2-(4'-t.-butyldimethylsilyloxyphenyl)-4H-1,3-benzoxazin

3 mmol 4-Adamantyliden-2-(4'-hydroxyphenyl)-4H-1,3-benzoxazin werden in 30 ml abs Methylenchlorid suspendiert. Es werden nacheinander 6 mmol Imidazol und 6 mmol t. -Butyldimethysilylchlorid zugegeben. Nach 14 h Rühren bei Raumtemp. wird filtriert. Das Filtrat wird nacheinander mit je 20 ml 1 M Natronlauge, 20 ml 2 M Salzsäure und 20 ml Wasser ausgeschüttelt. Die abgetrennte organische Phase wird über Natriumsulfat getrocknet Das Lösungsmittel wird abdestilliert. Der ölige Rückstand wird aus wenig Petroläther umkristallisiert.
Ausbeute 2.7 mmol

Wasser ausgeschüttelt. Die abgetrennte organische Phase wird über Natriumsulfat getrocknet Das Lösungsmittel wird abdestilliert. Der ölige Rückstand wird aus wenig Petroläther umkristallisiert.
Ausbeute 2.7 mmol

### Beispiel 5

### Dinatriumsalz von 4-Adamantyliden-2-(4'-phosphoryl-phenyl)-4H-1,3-benzoxazin

Eine Lösung von 3 mmol 4-Adamantyliden-2-(4'-hydroxyphenyl)-4H-1,3-benzoxazin in einer Mischung von 3 ml Aceton und 3 ml Pyridin wird unter Rühren bei 0 °C zu einer Lösung von 1.5 ml Phosphoroxychlorid in 7.5 ml Aceton getropft. Es wird 1 h bei 0°C gerührt und anschließend auf 60 ml gesättigte Kochsalzlösung gegossen und abgesaugt. Der Rückstand wird in 1.5 ml Wasser suspendiert. Mit 2 M Natronlauge wird ein pH-Wert von 10 eingestellt. Es wird mit 30 ml Ethanol versetzt und abgesaugt. Der Rückstand wird im Vakuum getrocknet.
Ausbeute 2.2. mmmol

### Beispiel 6

### Dinatriumsalz des 3-[2'-(4"-phosphoryl-phenyl)-4'H-1,3-benzoxazin-4'-yl]-4-spiroadamantyldioxetan

Unter Einleitung von trockenem Sauerstoff werden 2 mmol des Dinatriumsalzes von 4-Adamantyliden-2-(4'-phosphoryl-pheny)1-4H-1,3-benzoxazin gelöst in 20 ml abs. Methanol in Gegenwart von 0.01 mol% immobilisiertem Rose Bengal 2 h bei - 30 °C mit einer 1000 Watt Natriumdampflampe bestrahlt. Die Umsetzung verläuft quantitativ. Der Sensitizer läßt sich durch Abfiltrieren entfernen. Das Filtrat enthält das Dioxetan.
Ausbeute 2 mmol

### Beispiel 7:

### 2-(3'-Methoxyphenyl)-5,5-dimethyl-oxazolin-4-on

Zu einer Lösung von 57 mmol Kaliumcyanid in 50 ml Wasser werden bei 0 °C 50 mmol Aceton gegeben. 57 mmol 3-Methoxybenzoylchlorid werden innerhalb 90 min unter kräftigem Rühren zugetropft. Nach weiteren 90 min Rühren bei 0 °C wird 10 h bei Raumtemp stehengelassen. Es wird mit 100 ml Diethylether versetzt und die Mischung zweimal mit je 50 ml gesättigter Natriumhydrogencarbonatlösung und dann mit 100 ml Wasser ausgeschüttelt. Die organische Phase wird abgetrennt und über Natriumsulfat getrocknet. Das Lösungsmittel wird abdestilliert. Der ölige Rückstand (6.6 g 3-Methoxybenzoesäure-2'-cyanoisopropylester) wird im Hochvakuum getrocknet und anschließend in eine Mischung von 52 ml Acetanhydrid mit 10.4 ml 35 % wäßriger Tetrafluorborsäure eingetragen. Es wir 1 h unter Rückfluß zum Sieden erhitzt und nach Abkühlen auf Raumtemp. mit 300 ml Ether versetzt. Der entstandene Niederschlag wird in 70 ml Toluol suspendiert. Zu der Suspension werden 70 mmol Triethylamin gegeben und 1 h bei Raumtemp. gerührt. Es wird abfiltriert. Das Filtrat wird bis zur Trockene im Vakuum eingedampft. Der ölige Rückstand wird säulenchromatographisch (Kieselgel: Essigester /Petroläther 1 : 1) getrennt
Ausbeute 17 mmol

### Beispiel 8

### 4-Adamantyliden-2-(3'-methoxyphenyl)-5,5-dimethyl-oxazolin

Zu einer Suspension von 35 mmol Titantrichlorid in 60 ml abs. THF werden unter kräftigen Rühren vorsichtig 122 mmol Zink-Kupfer-Couple gegeben. Nach einstündigem Erhitzens unter Rückfluß wird nach Abkühlen auf Raumtemp. eine Mischung von 11.4 mmol 2-(3'-Methoxyphenyl)-5,5-dimethyl-oxazolin-4-on und 8.1 mmol Adamantanon gegeben. Es wird 4 h unter Rückfluß zum Sieden erhitzt. Nach Abkühlen auf Raumtemp. wird auf 200 ml Wasser gegossen. Die Mischung wird zweimal mit je 200 ml Essigester ausgeschüttelt. Die vereinigten organischen Phasen werden einmal mit Wasser gewaschen und abgetrennt. Nach Trocknen über Natriumsulfat wird filtriert und das Lösungsmittel abdestilliert. Der ölige Rückstand wird säulenchromatographisch (Kieselgel: Essigester/Petroläther 1: 9) getrennt.
Ausbeute 7 mmol

### Beispiel 9

### 4-Adamantyliden-2-(3'-hydroxyphenyl)-5,5-dimethyl-oxazolin

Unter Rühren wird bei -25°C unter Luft und Wasserausschluß eine Lösung von 7 mmol 4-Adamantyliden-2-(3'-methoxyphenyl)-5,5-dimethyl-oxazolin in 20 ml abs. Methylenchlorid zu einer Lösung von 14 mmol Bortrijodid getropft. Die Mischung wird innerhalb einer Stunde auf Raumtemp. erwärmt und weitere 4 h gerührt. Nach Versetzten mit 50 ml Wasser wird die organische Phase abgetrennt und über Natriumsulfat getrocknet. Das Lösungsmittel wird abdestilliert. Der ölige Rückstand wird säulenchromatographisch (Kieselgel: Essigester/Petroläther 1 : 3) getrennt.
Ausbeute 3.9 mmol

### Beispiel 10

### 4-Adamantyliden-2-(3'-t.butyldimethylsilyloxyphenyl)-5,5-dimethyl-oxazolin

3 mmol 4-Adamantyliden-2-(3'-hydroxyphenyl)-5,5-dimethyl-oxazolin werden in 30 ml abs Methylenchlorid suspendiert. Es werden nacheinander 6 mmol Imidazol und 6 mmol t. -Butyldimethysilylchlorid zugegeben. Nach 14 h Rühren bei Raumtemp. wird filtriert. Das Filtrat wird nacheinander mit je 20 ml 1M-Natronlauge, 20 ml 2 M-Salzsäure und 20 ml Wasser ausgeschüttelt. Die abgetrennte organische Phase wird über Natriumsulfat getrocknet. Das Lösungsmittel wird abdestilliert. Der ölige Rückstand wird säulenchromatographisch (Kieselgel:Petroläther) getrennt.
Ausbeute 2. 5 mmol

### Beispiel 11

### Dinatriumsalz von 4-Adamantyliden-2-(3'phosphorylphenyl)-5,5-dimethyl-oxazolin

Eine Lösung von 3 mmol 4-Adamantyliden-2-(3'-hydroxyphenyl)-5,5-dimethyl-oxazolin in einer Mischung von 3 ml Aceton und 3 ml Pyridin wird unter Rühren bei 0°C zu einer Lösung von 1.5 ml Phosphoroxychlorid in 7.5 ml Aceton getropft. Es wird 1 h bei 0°C gerührt und anschließend auf 60 ml gesättigte Kochsalzlösung gegossen und abgesaugt. Der Rückstand wird in 1.5 ml Wasser suspendiert. Mit 2M-Natronlauge wird ein pH-Wert von 10 eingestellt. Es wird mit 30 ml Ethanol versetzt und abgesaugt. Der Rückstand wird im Vakuum getrocknet.
Ausbeute 2.0 mmol

### Beispiel 12

### Dinatriumsalz des 3-[2'-(3"-Phosphorylphenyl)-5',5'-dimethyl-oxazolin-4'-yl}-4-spiroadamantyl-dioxetan

Unter Einleitung von trockenem Sauerstoff werden 2 mmol Dinatriumsalz von 4-Adamantyliden-2-(3'phosphorylphenyl)-5,5-dimethyl-oxazolin gelöst in 20 ml abs. Methanol in Gegenwart von 0.01 mol% immobilisiertem Rose Bengal 2 h bei - 30 °C mit einer 1000 Watt Natriumdampflampe bestrahlt. Die Umsetzung verläuft quantitativ. Der Sensitizer läßt sich durch Abfiltrieren entfernen. Das Filtrat enthält das Dioxetan.
Ausbeute 2 mmol

### Beispiel 13

### 7-Hydroxy-2,2-dimethyl-benzofuran-3-on

7-Hydroxy-2,3-dihydro-2,2-dimethylbenzofuran werden in 100 ml Pyridin gelöst. Bei 0°C wird innerhalb von 4 h 0.5 mol Benzoylchlorid zugetropft. Anschließend wird 10 h bei Raumtemperatur gerührt. Es wird auf 200 ml Eiswasser gegossen und abgesaugt. Der Rückstand wird mit 500 ml 2 M Salzsäure und dann mit 1000 ml Wasser gewaschen und an der Luft getrocknet. Zu einer Lösung dieses Rohprodukts (0.45 mol) wird in 600 ml Eisessig wird eine Lösung von 0.906 mol Chromtrioxid in 370 ml Eisessig so zugetropft, daß die Temperatur der Mischung 20 °C nicht überschreitet. Nach beendetem Zutropfen wird noch 1 h bei 40 °C gerührt und anschließend auf 21 Wasser gegossen. Es wird abgesaugt. Der Rückstand wird mit 1 L Wasser gewaschen und aus Ethanol/ Wasser 3 : 1 umkristallisiert. Die Benzoylverbindung wird in 400 ml 10 % ethanolische Kaliumhydroxidlösung eingetragen und die Mischung 1 h auf 70°C erwärmt. Es wird mit 3 1 Wasser versetzt und abgesaugt. Der Rückstand wird aus 2 1 Wasser umkristallisiert.
Ausbeute 0.16 mol

### Beispiel 14

### 3-(5'-Chlor-tricyclo [3.3.1.1.^{3,7}]yl)-7-hydroxy-2,2-dimethyl-benzofuran

Zu einer Suspension von 158 mmol Titantrichlorid in 120 ml abs. THF werden unter kräftigen Rühren vorsichtig 550 mmol Zink-Kupfer-Couple gegeben. Nach einstündigem Erhitzens unter Rückfluß wird bei Raumtemp. eine Mischung von 52 mmol 7-Hydroxy-2,2-dimethyl-benzofuran-3 on und 37 mmol 5-Chloradamantan-2-on gegeben. Es wird 30 min unter Rückfluß zum Sieden erhitzt. Nach Abkühlen auf Raumtemp. wird auf 200 ml Wasser gegossen. Die Mischung wird zweimal mit je 200 ml Essigester ausgeschüttelt. Die vereinigten organischen Phasen werden einmal mit Wasser gewaschen und abgetrennt. Nach Trocknen über Natriumsulfat wird filtriert und das Lösungsmittel abdestilliert. Der ölige Rückstand wird säulenchromatographisch (Kieselgel: Essigester/Petroläther 1 : 9) getrennt.
Ausbeute 30 mmol

### Beispiel 15

### 3-(5'-Chlor-tricyclo [3.3.1.1.^{3,7}]yl)-7-t.-butyldimethylsilyloxy-2,2-dimethyl-benzofuran

30 mmol 3-(5'-Chlor-tricyclo [3.3.1.1.^{3,7}]yl)-7-hydroxy-2,2-dimethyl-benzofuran werden in 30 ml abs Methylenchlorid suspendiert. Es werden nacheinander 60 mmol Imidazol und 60 mmol t. -Butyldimethysilylchlorid zugegeben. Nach 14 h Rühren bei Raumtemp. wird filtriert. Das Filtrat wird nacheinander mit je 100 ml 1M-Natronlauge, 100 ml 1M-Salzsäure und 20 ml Wasser ausgeschüttelt. Die abgetrennte organische Phase wird über Natriumsulfat getrocknet Das Lösungsmittel wird abdestilliert. Der ölige Rückstand wird säulenchromatographisch (Kieselgel:Petroläther) getrennt.
Ausbeute 27 mmol

### Beispiel 16

### Dinatriumsalz von 3-(5'-Chlor-tricyclo [3.3.1.1.^{3,7}]yl)-7-phosphoryl-2,2-dimethyl-benzofuran

Eine Lösung von 30 mmol 3-(5'-Chlor-tricyclo [3.3.1.1.^{3,7}]yl)-7-hydroxy-2,2-dimethyl-benzofuran in einer Mischung von 30 ml Aceton und 30 ml Pyridin wird unter Rühren bei 0°C zu einer Lösung von 15 ml Phosphoroxychlorid in 75 ml Aceton getropft. Es wird 1 h bei 0°C gerührt und anschließend auf 600 ml gesättigte Kochsalzlösung gegossen und abgesaugt. Der Rückstand wird in 15 ml Wasser suspendiert. Mit 2M-Natronlauge wird ein pH-Wert von 10 eingestellt. Es wird mit 300 ml Ethanol versetzt und abgesaugt. Der Rückstand wird im Vakuum getrocknet.
Ausbeute 23 mmmol

### Beispiel 17

### 3-(7'-t.-Butyldimethylsilyloxy-2',2'-dimethyl-benzofuran-3'-yl) -4-spiro-(5"-Chlor-tricyclo [3.3.1.1.^{3,7}]yl)-1,2-dioxetan

Unter Einleitung von trockenem Sauerstoff werden 10 mmol 3-(5'-Chlor-tricyclo [3.3.1.1.^{3,7}]yl)-7-t.-butyldimethylsilyloxy-2,2-dimethyl-benzofuran gelöst in 200 ml abs. Methylenchlorid in Gegenwart von 0.01 mol% immobilisiertem Rose Bengal 2 h bei - 30 °C mit einer 1000 Watt Natriumdampflampe bestrahlt. Die Umsetzung verläuft quantitativ. Der Sensitizer läßt sich durch Abfiltrieren entfernen. Das Filtrat enthält das Dioxetan.
Ausbeute 20 mmol

### Beispiel 18

### Dinatriumsalz des 3-(7'-Phosphoryl-2',2'-dimethyl-benzofuran-3'-yl) -4-spiro-(5"-Chlor-tricyclo [3.3.1.1.^{3,7}]yl)-1,2-dioxetan

Unter Einleitung von trockenem Sauerstoff werden 2 mmol Dinatriumsalz von 3-(5'-Chlortricyclo [3.3.1.1.^{3,7}]yl)-7-phosphoryl-2,2-dimethyl-benzofuran gelöst in 20 ml abs. Methanol in Gegenwart von 0.01 mol% immobilisiertem Rose Bengal 2 h bei - 30 °C mit einer 1000 Watt Natriumdampflampe bestrahlt. Die Umsetzung verläuft quantitativ. Der Sensitizer läßt sich durch Abfiltrieren entfernen. Das Filtrat enthält das Dioxetan.
Ausbeute 2 mmol

### Beispiel 19

### 5-Hydroxy-2,2-dimethyl-benzofuran-3-on

80 mmol t-Butylhydrochinon werden zusammen mit 80 mmol Kaliumbromat in 100 ml Wasser aufgeschlämmt. Dazu werden unter Rühren 20 Tropfen 1M-Schwefelsäure gegeben. Die Reaktionsmischung wird langsam unter Rühren auf 80 °C erhitzt. Es entsteht eine klare orange Lösung des Chinons. Beim Abkühlen auf Raumtemperatur fallen orange Nadeln (t-Butylbenzochinon) aus. Diese werden noch feucht in 400 ml Ethanol gelöst. Die Lösung wird bei 5 °C 15 h mit einer 250 W- Natriumdampflampe bestrahlt (DC-Kontrolle). Das Lösungsmittel wird abdestilliert, der Rückstand mit Toluol/Hexan 1 : 1 gewaschen. Der zurückbleibende farblose kristalline Rückstand (2-(2'-Ethyl-2'-ethoxy-propyl-)-hydrochinon) wird in eine Mischung von 4 ml Ethanol mit 80 ml Eisessig eingetragen. Unter Rühren werden 4 ml 40 % wäßrige Tetrafluorborsäure zugegeben. Die Mischung wird auf 80 °C erhitzt, wobei eine klare Lösung entsteht. Nach Abkühlen auf Raumtemperatur wird auf 200 ml Wasser gegossen und mit Toluol extrahiert. Die organische Phase wird einmal mit Wasser gewaschen und über Kaliumcarbonat getrocknet. Nach Abdestillieren des Lösungsmittels bleibt ein Öl zurück. (5-Hydroxy-2,2-dimethyl-2,3-dihydro-benzofuran). Die Benzoylierung, Oxidation und Verseifung erfolgt wie oben unter Beispiel 15 beschrieben.
Ausbeute 25 mmol

### Beispiel 20

### 6-Chlor-5-hydroxy-2,2-dimethyl-benzofuran-3-on

25 mmol 7-Hydroxy-2,2-dimethyl-benzofuran-3-on wird mit 30 g Kieselgel in 200 ml Chloroform gerührt. Es wird eine Lösung von 25 mmol N-Chlor-diisopropylamin in 100 ml Chloroform (hergestellt aus Diisopropylamin und wäßriger Natriumhypochloridlösung, extrahiert in Chloroform, Konzentration iodometrisch bestimmt) zugetropft. Nach 10 h Rühren wird filtriert und das Filtrat mit Wasser ausgeschüttelt. Die Lösung wird über Natriumsulfat getrocknet. Das Lösungsmittel wird bei 300 mbar abdestilliert. Der Rückstand wird säulenchromatographisch (Kieselgel: Essigester) getrennt
Ausbeute 20 mmol

### Beispiel 21

### 6-Chlor-5-hydroxy-2,2-dimethyl-benzofuran-3-thion

Eine Lösung von 20 mmol 6-Chlor-5-hydroxy-2,2-dimethyl-benzofuran-3-on in 30 ml Toluol wird mit 10 mmol Lawesson's-Reagenz versetzt und 3 Stunden unter Rückfluß zum Sieden erhitzt. Nach Abkühlen auf Raumtemperatur wird über 50 g Kieselgel filtriert. Die rote Fraktion des Filtrats wird aufgefangen. Nach Abdestillieren des Toluols bleibt ein rotes Öl zurück.
Ausbeute 17 mmol

### Beispiel 22

### 3-((E/Z)-)-6-Chlor-5-hydroxy-2,2-dimethyl-benzofuran-3-yliden)-androsta-1,4-dien-17-on

Unter Rühren bei Raumtemperatur wird 1 mmol Androsta-1-4-dien-3,17-dion-3-hydrazon zu einer Suspension von 1.5 g Mangandioxid und 1.5 g Aluminiumoxid in 20 ml Ether, der mit 10 Tropfen Wasser versetzt wurde, eingetragen. Nach einstündigem Rühren wird die etherische Lösung des Diazosteroids abfiltriert. Zum Filtrat wird portionsweise 1 mmol 6-Chlor-5-hydroxy-2,2-dimethyl-benzofuran-3-thion gegeben. Nach zweistündigem Rühren wird der Ether abdestilliert und der Rückstand über Kieselgel (Essigester/Petroläther 1 : 9) chromatographiert. Die schwach gelb gefärbten Fraktionen werden gesammelt. Nach Abdestillieren des Lösungsmittels bleibt ein leicht gelber, kristalliner Rückstand zurück.
Ausbeute 0.7 mmol

### Beispiel 23

### 3-((E/Z)-)-6-Chlor-5-t-butyldimethylsilyloxy-2,2-dimethyl-benzofüran-3-yliden)-androsta-1,4-dien-17-on

30 mmol 3-(Androsta-1',4'-dien-17'-on-3'-yliden)-6-Chlor-5-hydroxy-2,2-dimethyl-benzofuran werden in 30 ml abs Methylenchlorid suspendiert. Es werden nacheinander 60 mmol Imidazol und 60 mmol t. -Butyldimethysilylchlorid zugegeben. Nach 14 h Rühren bei Raumtemp. wird filtriert. Das Filtrat wird nacheinander mit je 100 ml 1M-Natronlauge, 100 ml 1M-Salzsäure und 20 ml Wasser ausgeschüttelt. Die abgetrennte organische Phase wird über Natriumsulfat getrocknet Das Lösungsmittel wird abdestilliert. Der ölige Rückstand wird säulenchromatographisch (Kieselgel:Petroläther) getrennt.
Ausbeute 24 mmol

### Beispiel 24

### 3-((E/Z)-)-6'-Chlor-2',2'-dimethyl-benzofuran-3'-yliden)-androsta-1,4-dien-17-on-5'-yldinatriumphosphat

Eine Lösung von 30 mmol 3-(Androsta-1',4'-dien-17'-on-3'-yliden)-3-Chlor-5-hydroxy-2,2-dimethyl-benzofuran in einer Mischung von 30 ml Aceton und 30 ml Pyridin wird unter Rühren bei 0°C zu einer Lösung von 15 ml Phosphoroxychlorid in 75 ml Aceton getropft. Es wird 1 h bei 0°C gerührt und anschließend auf 600 ml gesättigte Kochsalz gegossen und abgesaugt. Der Rückstand wird in 15 ml Wasser suspendiert. Mit 2M-Natronlauge wird ein pH-Wert von 10 eingestellt. Es wird mit 300 ml Ethanol versetzt und abgesaugt. Der Rückstand wird im Vakuum getrocknet.
Ausbeute 23 mmol

### Beispiel 25

### (4'Ξ)-5 "-t.-Butyldimethylsilyloxy-6"-Chlor-2",2"-dimethyl-17-oxo-(3ξO)-dispiro [androsta-1,4-dien-3,3'-[1,2]dioxetan-4',3"-benzofuran

Unter Einleitung von trockenem Sauerstoff werden 10 mmol 3-(Androsta-1',4'-dien-17'-on-3'-yliden)-6-Chlor-5-t-butyldimethylsilyloxy-2,2-dimethyl-benzofuran gelöst in 20 ml abs. Methylenchlorid in Gegenwart von 0.01 mol% immobilisiertem Rose Bengal 2 h bei - 30 °C mit einer 1000 Watt Natriumdampflampe bestrahlt. Die Umsetzung verläuft quantitativ. Der Sensitizer läßt sich durch Abfiltrieren entfernen. Das Filtrat enthält das Dioxetan.
Ausbeute 20 mmol

### Beispiel 26

### (4'Ξ)-6"-Chlor-2",2"-dimethyl-17-oxo-(3ξO)-dispiro[androsta-1,4-dien-3,3'-[1,2]dioxetan-4',3"-benzofuran-5"-yl-dinatriumphosphat

Unter Einleitung von trockenem Sauerstoff werden 2 mmol Dinatriumsalz von 3-(Androsta-1',4'-dien-17'-on-3'-yliden)-6-Chlor-5-phosphoryl-2,2-dimethyl-benzofuran gelöst in 20 ml abs. Methanol in Gegenwart von 0.01 mol% immobilisiertem Rose Bengal 8 h bei - 30 °C mit einer 1000 Watt Natriumdampflampe bestrahlt. Die Umsetzung verläuft quantitativ. Der Sensitizer läßt sich durch Abfiltrieren entfernen. Das Filtrat enthält das Dioxetan.
Ausbeute 2 mmol

Analog bis zu den Beispielen 1 bis 28 konnten die entsprechenden Dioxetane auf Basis der folgenden Benzofuranderivate hergestellt werden:
5-Hydroxy-2,2-dimethyl-2,3-dihydro-benzofuran,
5-Hydroxy-2,2-dimethyl-2,3-dihydro-benzofuran-3-on,
5-Hydroxy-2,2-dimethyl-2,3-dihydro-benzofuran-3-thion,
6-Chlor-5-hydroxy-2,2-dimethyl-benzofuran-3-on sowie
6-Chlor-5-hydroxy-2,2-dimethyl-benzofuran-3-thion.

## Patentansprüche

1. Verbindungen der allgemeinen Formel Ia, 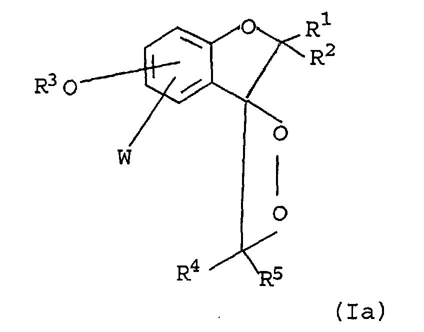 in der
R¹ und R² gleich oder verschieden sind und Wasserstoff, Niederalkyl C₁ bis C₆, geradkettig oder verzweigt, jeweils oder zusammen eine Phenylgruppe darstellen, mit der Maßgabe, daß höchstens einer der Reste R¹ oder R² Wasserstoff ist,
der Rest -O-R³ das entsprechende Hydroxysalz, eine Oxysäure, Phosphat, Aryl- oder Alkylcarboxylester, Alkyloxy, Trialkyloxy oder Alkylsilyloxy, Arylsilyloxy, Sulfat, Oxypyranosid oder ein Glyceridyl- bzw. Phosphorylrest oder ein Steroidderivat darstellt,
W Wasserstoff, Halogen oder ein Rhodanid- oder Cyanidrest,
R⁴ oder R⁵ Wasserstoff oder eine die Dioxetanstruktur stabilisierende Gruppe darstellt, wobei höchstens eine der Gruppen R⁴ oder R⁵ Wasserstoff ist und die dioxetanstabilisierende Gruppe ein Adamantyl-, Phenyl-, Cylohexyl-, sekundärer oder tertiärer Alkyl-, Polycycloalkyl- bzw. Polycycloarylrest, ein Steroidderivat oder ein Gemisch von letzteren ist.

2. Verbindungen nach Anspruch 1, **dadurch gekennzeichnet, daß** die Reste R¹ oder R² Wasserstoff, eine Methyl-, Ethyl- oder eine Phenylgruppe bedeutet.

3. Verbindungen nach Anspruch 1 oder 2, **dadurch gekennzeichnet, daß** es sich um 3-(7'-t.-Butyldimethylsilyloxy-2',2'-dimethyl-benzofuran-3'-yl)-4-spiro-(tricyclo [3.3.1.1.^{3,7}]yl)-1,2-dioxetan, das Dinatriumsalz des 3-(7'-Phosphoryl-2',2'-dimethyl-benzofuran-3'-yl)-4-spiro-(tricyclo[3.3.1.1.^{3,7}]yl)-1,2-dioxetans, 3-(7'-t.-Butyldimethylsilyloxy-2',2'-dimethyl-benzofuran-3'-yl)-4-spiro-(5"-chlor-tricyclo[3.3.1.1.^{3,7}]yl)-1,2-dioxetan, das Dinatriumsalz des 3-(7'-Phosphoryl-2',2'-dimethyl-benzofuran-3'-yl)-4-spiro-(5"-chlor-tricyclo [3.3.1.1.^{3,7}]yl)-1,2-dioxetans, 3-(5'-t.-Butyldimethylsilyloxy-2',2'-dimethyl-benzofuran-3'-yl) -4-spiro-(5"chlor-tricyclo[3.3.1.1.^{3,7}]yl)-1,2-dioxetan, das Dinatriumsalz des 3-(5'-Phosphoryl-2',2'-dimethyl-benzofuran-3'-yl)-4-spiro-(5"-Chlor-tricyclo[3.3.1.1.^{3,7}]yl)-1,2-dioxetans, 3-(5'-t.-Butyldimethylsilyloxy-6'-chlor-2',2'-dimethyl-benzofuran-3'-yl) -4-spiro-(5"-chlor-tricyclo [3.3.1.1.^{3,7}]yl)-1,2-dioxetan, das Dinatriumsalz des 3-(5'-Phosphoryl-6'-chlor-2',2'-dimethylbenzofuran-3'-yl)-4-spiro-(5"-chlor-tricyclo[3.3.1.1.^{3,7}]yl)-1,2-dioxetans, (4'Ξ)-5"-t.-Butyldimethylsilyloxy-6"-Chlor-2",2"-dimethyl-17-oxo-(3ξO)-dispiro[androsta-1,4-dien-3,3'-[1,2]dioxetan-4',3"-benzofuran oder (4'Ξ)-6"-Chlor-2",2"-dimethyl-17-oxo-(3ξO)-dispiro [androsta-1,4-dien-3,3'-[1,2]dioxetan-4',3"-benzofuran-5"-yl-dinatriumphosphat handelt.

4. Verfahren zur Herstellung von Verbindungen der allgemeinen Formel Ia 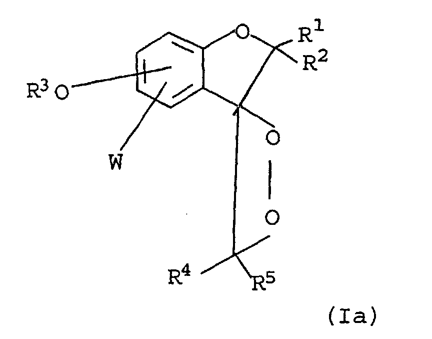 **dadurch gekennzeichnet, daß** eine der Verbindungen gemäß der allgemeinen Formel IV 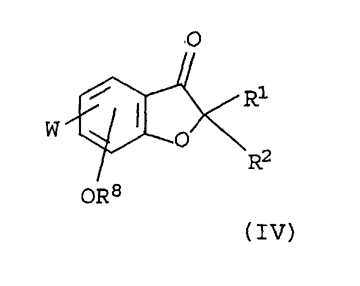 mit einer Verbindung der allgemeinen Formel V 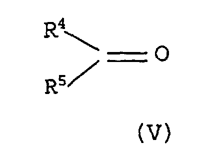 worin die Reste R¹ bis R⁵, W, X die jeweils bereits angegebenen Bedeutungen haben und R⁸ einen Alkylrest, geradkettig oder verzweigt mit 1 bis 6 C-Atomen, oder Wasserstoff darstellt, in Gegenwart von Titantrichlorid und einem Reduktionsmittel umgesetzt werden.

5. Verfahren nach Anspruch 4, **dadurch gekennzeichnet, daß** als Reduktionsmittel Zink/Kupfer oder Lithiumaluminiumhydrid verwendet wird.

6. Verfahren zur Herstellung von Verbindungen der allgemeinen Formel Ia 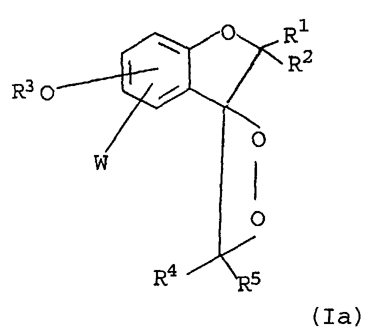 **dadurch gekennzeichnet, daß** eine der Verbindungen der allgemeinen Formel IV 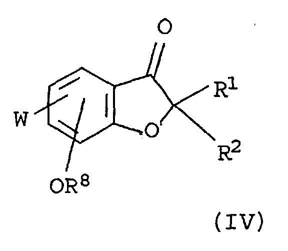 zunächst in die entsprechende Thioncarbonylverbindung überführt wird und anschließend mit einer Diazoverbindung der allgemeinen Formel IX 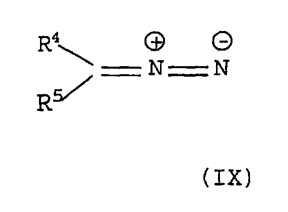 worin die Reste R¹ bis R⁵, R⁸, W, X die jeweils bereits angegebenen Bedeutungen haben, umgesetzt, dealkyliert, die abspaltbare Gruppe R³ eingeführt und dioxygeniert wird.

7. Verfahren nach Anspruch 6, **dadurch gekennzeichnet, daß** die Umwandlung der Carbonylin die Thiocarbonylfunktion in Gegenwart eines Schwefelreagenzes oder von Phosphopentasulfid erfolgt.

8. Verfahren nach Anspruch 6 oder 7, **dadurch gekennzeichnet, daß** man als Diazoverbindung, Diazoadamantan, Chlordiazoadamantan, Diphenyldiazomethan, polycyclische Diazoverbindungen, sekundäre oder tertiäre Ketone bzw. Aldehyde, die gegebenenfalls substituiert sein können, verwendet.

9. Verfahren zur Bestimmung einer Säure, Base, eines Salzes, Enzyms, anorganischen oder organischen Katalysators oder Elektronendonors durch Umsetzung dieses Analyten mit einer Verbindung gemäß Anspruch 1, Abspaltung der Gruppe R³ und Messung des emittierten Lichts als Maß für den Gehalt an der zu bestimmenden Verbindung.

10. Verwendung der Verbindungen nach den Ansprüchen 1 bis 3 in in-vitro immunologischen Assays oder zur in vitro DNA-Diagnostik.

## Claims

1. Compounds of the general formula Ia 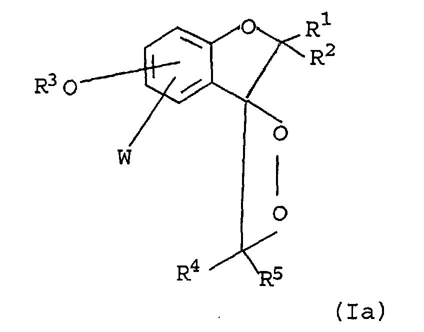 in which
R¹ and R² are the same or different and represent hydrogen, straight-chained or branched C₁ to C₆ lower alkyl, or individually or together represent a phenyl group provided that no more than one of the residues R¹ or R² is hydrogen,
the residue O-R³ represents the corresponding hydroxy salt, an oxy acid, phosphate, aryl or alkyl carboxylic ester, alkyloxy, trialkyloxy or alkylsilyloxy, arylsilyloxy, sulfate, oxypyranoside or a glyceridyl or phosphoryl residue or a steroid derivative,
W represents hydrogen, halogen or a thiocyanate or cyanide residue,
R⁴ or R⁵ represents hydrogen or a group stabilizing the dioxetane structure, where no more than one of the groups R⁴ or R⁵ is hydrogen and the dioxetane-stabilizing group is an adamantyl, phenyl, cyclohexyl, secondary or tertiary alkyl, polycycloalkyl or polycycloaryl residue, a steroid derivative or a mixture of the latter.

2. Compounds as claimed in claim 1, **characterized in that** the residues R¹ or R² denote hydrogen, a methyl, ethyl or phenyl group.

3. Compounds as claimed in claim 1 or 2, **characterized in that** they are 3-(7'-t.-butyldimethylsilyloxy-2',2'-dimethyl-benzofuran-3'-yl)-4-spiro-(tricyclo[3.3.1.1.^{3,7}] yl)-1,2-dioxetane, the disodium salt of 3-(7'-phosphoryl-2',2'-dimethyl-benzofuran-3'-yl)-4-spiro-(tricyclo [3.3.1.1.^{3,7}]yl)-1,2-dioxetane, 3-(7'-t.-butyl-dimethylsilyloxy-2',2'-dimethyl-benzofuran-3'-yl)-4-spiro-(5"-chloro-tricyclo [3.3.1.1.^{3,7}]yl)-1,2-dioxetane, the disodium salt of 3-(7'-phosphoryl-2',2'-dimethyl-benzofuran-3'-yl)-4-spiro-(5"-chlorotricyclo[3.3.1.1.^{3,7}]-yl)-1,2-dioxetane, 3-(5'-t.-butyldimethylsilyloxy-2',2'-dimethyl-benzofuran-3'-yl)-4-spire-(5"-chloro-tricyclo[3.3.1.1.^{3,7}]yl)-1,2-dioxetane, the disodium salt of 3-(5'-phosphoryl-2',2'-dimethyl-benzofuran-3'-yl)-4-spiro-(5"-chloro-tricyclo [3.3.1.1.^{3,7}]yl)-1,2-dioxetane, 3-(5'-t.-butyldimethylsilyloxy-6'-chloro-2',2'-dimethyl-benzofuran-3'-yl)-4-spiro-(5"-chlorotricyclo [3.3.1.1.^{3,7}]yl)-1,2-dioxetane, the disodium salt of 3-(5'-phosphoryl-6'-chloro-2',2'-dimethyl-benzofuran-3'-yl)-4-spiro-(5"-chlorotricyclo[3.3.1.1.^{3,7}]yl)-1,2-dioxetane, (4'Ξ)-5"-t.-butyl-dimethylsilyloxy-6"-chloro-2",2"-dimethyl-17-oxo-(3ζO)-dispiro[androsta-1,4-diene-3,3'-[1,2]dioxetane-4',3"-benzofurane or (4'Ξ)-6"-chloro-2",2"-dimethyl-17-oxo-(3ξO)-dispiro [androsta-1,4-diene-3,3'-[1,2]dioxetane-4',3"-benzofuran-5"-yl-disodium phosphate.

4. Process for the production of compounds of the general formula Ia 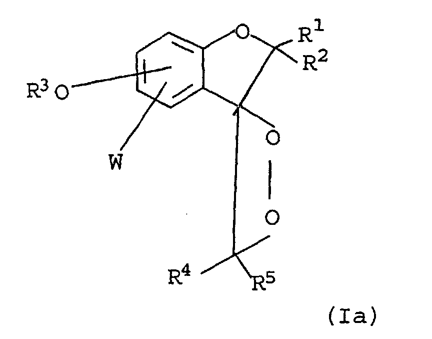 **characterized in that** one of the compounds of the general formula IV 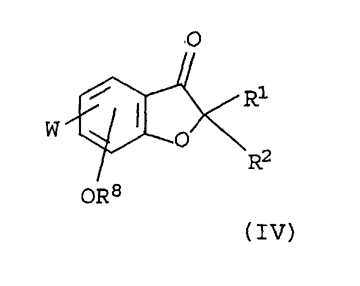 is reacted in the presence of titanium trichloride and a reducing agent with a compound of the general formula V 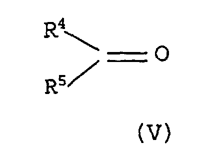 in which the residues R¹ to R⁵, W and X have the meanings already stated and R⁸ represents a straight-chained or branched alkyl residue with 1 to 6 C atoms or hydrogen.

5. Process as claimed in claim 4, **characterized in that** zinc/copper or lithium aluminium hydride is used as the reducing agent.

6. Process for the production of compounds of the general formula Ia 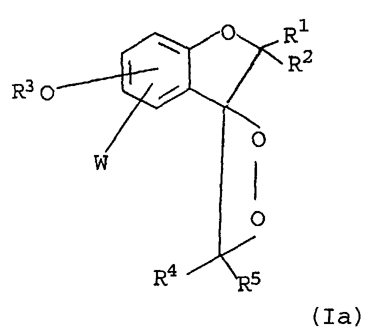 **characterized in that** one of the compounds of the general formula IV 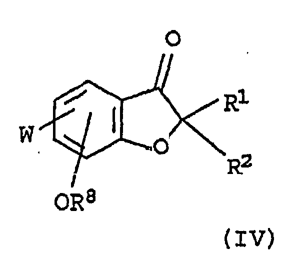 is firstly converted into the respective thiocarbonyl compound and subsequently reacted with a diazo compound of the general formula IX 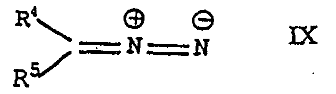 in which the residues R¹ to R⁵, R⁸, W and X have the meanings already stated, it is dealkylated, the cleavable group R³ is introduced and it is dioxygenated.

7. Process as claimed in claim 6, **characterized in that** the carbonyl group is converted into a thiocarbonyl group in the presence of a sulphur reagent or phosphopentasulphide.

8. Process as claimed in claim 6 or 7, **characterized in that** diazoadamantane, chlorodiazoadamantane, diphenyldiazomethane, polycyclic diazo compounds, secondary or tertiary ketones or aldehydes which can optionally be substituted are used as the diazo compound.

9. Method for the determination of an acid, base, salt, enzyme, inorganic or organic catalyst or electron donor by reacting this analyte with a compound according to claim 1, cleaving the group R³ and measuring the emitted light as a measure for the content of the compound to be determined.

10. Use of the compounds as claimed in one of the claims 1 to 3 in in-vitro immunological assays or for in-vitro DNA diagnostics.

## Revendications

1. Composés répondant à la formule générale la 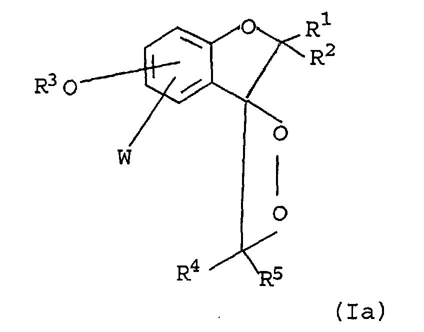 dans laquelle
R¹ et R² sont identiques ou différents et représentent un atome d'hydrogène, un groupe alkyle inférieur contenant de 1 à 6 atomes de carbone, à chaîne droite ou ramifiée, respectivement ou de manière conjointe un groupe phényle, avec cette mesure qu'au maximum un des radicaux R¹ ou R² représente un atome d'hydrogène ;
le radical -O-R³ représente l'hydroxysel correspondant, un oxyacide, un phosphate, un ester aryl- ou alkylcarboxylique, un groupe alkyloxy, un groupe trialkyloxy ou un groupe alkylsilyloxy, un groupe arylsilyloxy, un sulfate, un groupe oxypyranoside ou un radical glycéridyle, respectivement phosphoryle ou encore un dérivé stéroïdien,
W représente un atome d'hydrogène, un atome d'halogène ou encore un radical sulfocyanure ou un radical cyanure,
R⁴ ou R⁵ représente un atome d'hydrogène ou un groupe stabilisant la structure dioxétane, au maximum un des groupes R⁴ ou R⁵ représentant un atome d'hydrogène, et le groupe stabilisateur du groupe dioxétane représente un radical adamantyle, un radical phényle, un radical cyclohexyle, radical alkyle secondaire ou tertiaire, un radical polycycloalkyle, respectivement polycycloaryle, un dérivé stéroïdien, ou encore un mélange desdits radicaux.

2. Composé selon la revendication 1, **caractérisé en ce que** le radical R¹ ou R² représente un atome d'hydrogène, un groupe méthyle, un groupe éthyle ou un groupe phényle.

3. Composés selon la revendication 1 ou 2, **caractérisé en ce qu'**il s'agit du 3-(7'-tert.-butyldiméthylsilyloxy-2',2'-diméthyl-benzofuran-3'-yl)-4-spiro-(tricyclo[3.3.1.1^{3,7}]yl)-1,2-dioxétane, le sel disodique du 3-(7'-phosphoryl-2',2'-diméthyl-benzofuran-3'-yl)-4-spiro-(tricyclo-[3.3.1.1^{3,7}]yl)-1,2-dioxétane, le 3-(7'-tert.-butyldiméthylsilyloxy-2',2'-diméthylbenzofuran-3'-yl)-4-spiro-(5"-chloro-tricyclo[3.3.1.1^{3,7}]yl)-1,2-dioxétane, le sel disodique du 3-(7'-phosphoryl-2',2'-diméthyl-benzofuran-3'-yl)-4-spiro-(5"-chloro-tricyclo-[3.3.1.1^{3,7}]yl)-1,2-dioxétane, le 3-(5'-tert.-butyldiméthylsilyloxy-2',2'-diméthyl-benzofuran-3'-yl)-4-spiro-(5"-chlorotricyclo[3.3.1.1^{3,7}]yl)-1,2-dioxétane, le sel disodique du 3-(5'-phosphoryl-2',2'-diméthyl-benzofuran-3'-yl)-4-spiro-(5"-chloro-tricyclo-[3.3.1.1^{3,7}]yl)-1,2-dioxétane, le 3-(5'-tert.-butyldiméthylsilyloxy-6'-chloro-2',2'-diméthylbenzofuran-3'-y|)-4-spiro-(5"-chloro-tricyclo[3.3.1.1^{3,7}]yl)-1,2-dioxétane, le sel disodique du 3-(5'-phosphoryl-6'-chloro-2',2'-diméthyl-benzofuran-3'-yl)-4-spiro-(5"-chloro-tricyclo-[3.3.1.1^{3,7}]yl)-1,2-dioxétane, le (4'Ξ)-5"-tert.-butyldiméthylsilyloxy-6"-chloro-2",2"-diméthyl-17-oxo-(3ξO)-dispiro-[androsta-1,4-diène-3,3'-[1,2]dioxétane-4',3"-benzofurane ou le phosphate disodique du (4'Ξ)-6"-chloro-2",2"-diméthyl-17-oxo-(3ξO)-dispiro-[androsta-1,4-diène-3,3'-[1,2]dioxétane-4',3"-benzofuran-5"-yle.

4. Procédé pour la préparation de composés répondant à la formule générale la 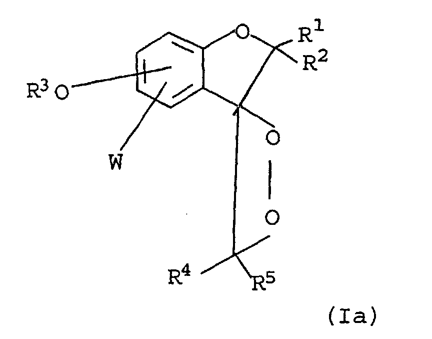 **caractérisé en ce qu'**on fait réagir un des composés répondant à la formule générale IV 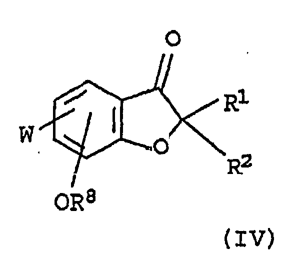 avec un composé répondant à la formule générale V 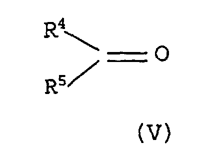 les radicaux R¹ à R⁵, W, X ayant respectivement les significations déjà indiquées et R⁸ représentant un radical alkyle à chaîne droite ou ramifiée contenant de 1 à 6 atomes de carbone ou encore un atome d'hydrogène, en présence de trichlorure de titane et d'un agent de réduction.

5. Procédé selon la revendication 4, **caractérisé en ce qu'**on utilise, à titre d'agent de réduction, du zinc/cuivre ou encore de l'hydrure de lithium-aluminium.

6. Procédé pour la préparation de composés répondant à la formule générale Ia 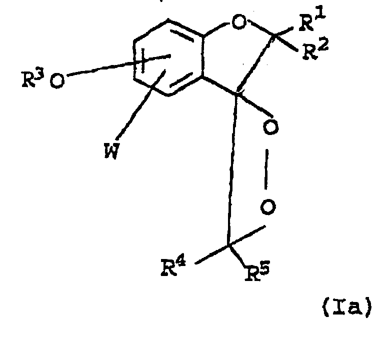 **caractérisé en ce qu'**on transforme d'abord un des composés répondant à la formule générale IV 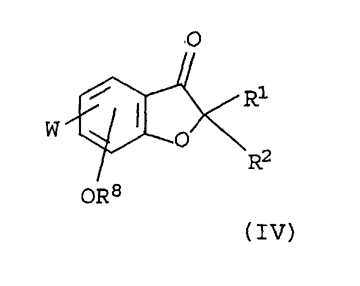 en composé thiocarbonyle correspondant avant de le faire réagir avec un composé diazoïque répondant à la formule générale IX 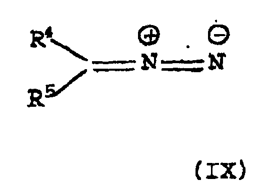 les radicaux R¹ à R⁵, R⁸, W, X ayant respectivement les significations déjà indiquées, et avant de procéder à une désalkylation, à l'introduction du groupe R3 apte à être éliminé et à une dioxygénation.

7. Procédé selon la revendication 6, **caractérisé en ce que** la transformation de la fonction carbonyle en fonction thiocarbonyle a lieu en présence d'un réactif à base de soufre ou d'un phosphopentasulfure.

8. Procédé selon la revendication 6 ou 7, **caractérisé en ce qu'**on utilise, à titre de composé diazoïque, le diazoadamantane, le chlorodiazoadamantane, le diphényldiazométhane, des composés diazoïques polycycliques, des cétones secondaires ou tertiaires, respectivement des aldéhydes, lesdits composés pouvant, le cas échéant, être substitués.

9. Procédé pour la détermination d'un acide, d'une base, d'un sel, d'une enzyme, d'un catalyseur inorganique ou organique ou encore d'un donneur d'électrons par mise en réaction de cet analyte avec un composé selon la revendication 1, élimination du groupe R³ et évaluation de la lumière d'émission comme mesure de la teneur du composé à déterminer.

10. Utilisation des composés selon les revendications 1 à 3 dans des dosages immunologiques in vitro ou pour le diagnostic d'ADN in vitro.
